# EUROPÄISCHE PATENTSCHRIFT

(11) **EP 1 529 025 B1**
(45) Veröffentlichungstag und Bekanntmachung des Hinweises auf die Patenterteilung: **16.07.2008**
(21) Anmeldenummer: 03784036.0
(22) Anmeldetag: 22.07.2003
(51) Int. Cl.: C07C 49/835, C07C 49/84, C07C 45/78, A61K 31/122, A61P 31/04

(54) **POLYISOPRENYL-BENZOPHENON-DERIVATE, VERFAHREN ZU IHRER HERSTELLUNG UND VERWENDUNG DERSELBEN**
POLYISOPRENYLBENZOPHENONE DERIVATIVES, METHOD FOR PRODUCTION AND USE THEREOF
DERIVES DE POLYISOPRENYL-BENZOPHENONE, PROCEDE DE PRODUCTION ET UTILISATION DE CES DERNIERS

(30) Priorität: 07.08.2002 DE 10236262
(43) Veröffentlichungstag der Anmeldung: 11.05.2005
(73) Patentinhaber: Sanofi-Aventis Deutschland GmbH, 65929 Frankfurt am Main (DE)
(72) Erfinder: SCHIELL, Matthias, 65611 Brechen (DE); KURZ, Michael, 65926 Frankfurt (DE); HAAG-RICHTER, Sabine, 65926 Frankfurt (DE)
(86) Internationale Anmeldenummer: PCT/EP2003/007965
(87) Internationale Veröffentlichungsnummer: WO 2004/014831

(56) Entgegenhaltungen:
- WO-A-03/043966
- LOKVAM J ET AL: "Two polyisoprenylated benzophenones from the trunk latex of Clusia grandiflora (Clusiaceae)" PHYTOCHEMISTRY, PERGAMON PRESS, GB, Bd. 55, Nr. 1, September 2000 (2000-09), Seiten 29-34, XP004291556 ISSN: 0031-9422 in der Anmeldung erwähnt

## Beschreibung

Zur Behandlung von bakteriellen Infektionskrankheiten wird eine große Zahl von Antibiotika eingesetzt. Die Krankheitserreger werden aber zunehmend resistent gegen die verwendeten Arzneimittel, und es droht eine große Gefahr durch sogenannte multiresistente Keime, die nicht nur gegen einzelne Antibiotikagruppen, wie z.B. β-Lactam-Antibiotika, Glycopeptide oder Macrolide widerstandsfähig geworden sind, sondern gleichzeitig mehrere Resistenzen tragen. Es gibt sogar Krankheitserreger, die gegen alle im Handel erhältlichen Antibiotika resistent geworden sind. Infektionskrankheiten, die durch solche Keime verursacht werden, sind nicht mehr therapierbar. Deshalb gibt es einen großen Bedarf an neuen Mitteln, die gegen resistente Keime eingesetzt werden können. Es sind zwar in der Literatur viele Tausend antibiotisch aktive Verbindungen beschrieben worden, die meisten sind jedoch zu toxisch, um als Arzneimittel eingesetzt werden zu können.

Es ist bereits eine Anzahl von Substanzen des Polyisoprenyl-benzophenon-Typ beschrieben worden. Es handelt sich dabei hauptsächlich um aus Pflanzen isolierte Substanzen.

Rama Rao et al. (Tetrahedron Lett., 1980, 21, 1975-1978) beschreiben das Camboginol und das Cambogin, die aus Garcinia gambogia isolierbar sind, und für die keine pharmakologische Wirkung beschrieben wurde.

Fuller et al. (J. Nat. Prod., 1999, 62, 130-132) beschreiben die Verbindung Guttiferone F, die HIV-inhibierend wirkt.

Lokvam et al. (Phytochemistry, 2000, 55, 29-34) beschreiben die Verbindungen Chamone I bzw. Nemorosome II als wirksam gegen bestimmte Bienen-pathogene Bakterien.

Aufgabe der vorliegenden Erfindung ist es, alternative Verbindungen vom Polyisoprenyl-benzophenon-Typ mit verbesserten pharmakologischen Wirkungen bereitzustellen.

Es wurde überraschend gefunden, daß die Pflanze Garcinia punctata aus der Familie der Clusiaceae neuartige Verbindungen zu bilden vermag, die insbesondere gegen human-pathogene Bakterien wirksam sind.

Die Erfindung betrifft daher eine Verbindung der Formel (I) 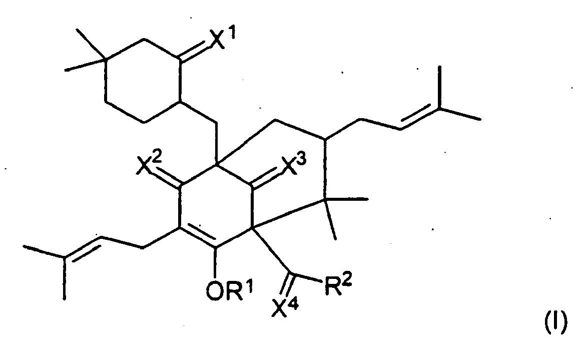 wobei
R¹
H, C₁-C₆-Alkyl, C₂-C₆-Alkenyl, C₂-C₆-Alkinyl oder C₆-C₁₄-Aryl bedeutet,
worin Alkyl, Alkenyl, Alkinyl und Aryl unsubstituiert oder ein- bis dreifach durch einen Rest R³ substituiert sind,
R²
C₁-C₆-Alkyl, C₂-C₆-Alkenyl, C₂-C₆-Alkinyl oder C₆-C₁₄-Aryl bedeutet,
worin Alkyl, Alkenyl, Alkinyl und Aryl unsubstituiert oder n-fach durch einen Rest R³ substituiert sind, wobei n eine ganze Zahl von 1 bis 3 ist, und
R³
-OH, =O, -O-C₁-C₆-Alkyl, -O-C₂-C₆-Alkenyl, -O-C₆-C₁₄-Aryl, -NH-C₁-C₆-Alkyl, -NH-C₂-C₆-Alkenyl, -NH[-C(=O)-(C₁-C₆-Alkyl)], -NH[-C(=O)-(C₆-C₁₄-Aryl)], -NH₂ oder Halogen bedeutet, wenn R¹ und R² unabhängig voneinander Alkyl, Alkenyl oder Alkinyl sind, worin Alkyl, Alkenyl und Alkinyl weiter substituiert sein können durch -CN, -Amid oder -Oxim-Funktionen, oder
-OH, -O-C₁-C₆-Alkyl, -O-C₂-C₆-Alkenyl, -O-C₆-C₁₄-Aryl, -NH-C₁-C₆-Alkyl, -NH-C₂-C₆-Alkenyl, -NH[-C(=O)-(C₁-C₆-Alkyl)], -NH[-C(=O)-(C₆-C₁₄-Aryl)], -NH₂ oder Halogen bedeutet, wenn R¹ oder R² Aryl sind, worin Aryl weiter substituiert sein kann durch -CN oder -Amid,
X¹
=CH₂ oder =O bedeutet,
X², X³ und X⁴ unabhängig voneinander
=O, =NR¹ oder =S bedeutet,
oder eine stereoisomere Form der Verbindung der Formel (I) oder ein Gemisch von Stereoisomeren einer Verbindnung der Formel (I) in jedem Verhältnis, oder ein physiologisch verträgliches Salz einer Verbindung der Formel (I) oder ein physiologisch verträgliches Salz einer stereoisomeren Form einer Verbindung der Formel (I).

C₁-C₆-Alkyl bedeutet ein geradkettiges oder verzweigtes Alkyl mit 1 bis 6 C-Atomen, bevorzugt mit 1 bis 4 C-Atomen, z.B. Methyl, Ethyl, i-Propyl, tert.Butyl und Hexyl.

C₂-C₆-Alkenyl bedeutet ein geradkettiges oder verzweigtes Alkenyl mit 2 bis 6 C-Atomen, das einfach, zweifach oder dreifach ungesättigt ist, z.B. Allyl, Crotyl, 1-Propenyl, Penta-1,3-dienyl und Pentenyl.

C₂-C₆-Alkinyl bedeutet ein geradkettiges oder verzweigtes Alkinyl mit 2 bis 6 C-Atomen, das einfach oder zweifach ungesättigt ist, z.B. Propinyl, Butinyl und Pentinyl.

C₆-C₁₄-Aryl bedeutet einen aromatische Rest mit 6 bis 14 C-Atomen, vorzugsweise 6 bis 10 C-Atomen, beispielsweise Phenyl,1- Naphthyl oder 2-Naphthyl, der unsubstituiert oder substituiert ist durch Halogen, C₁-C₄-Alkyl, vorzugsweise Methyl, Hydroxy, C₁-C₄-Alkoxy, vorzugsweise Methoxy, oder durch Trifluormethyl.

Aliphatische Acylgruppen -NH[-C(=O)-(C₁-C₆-Alkyl)] enthalten vorzugsweise eine C₁-C₄-Alkylgruppe, beispielsweise Formyl, Acetyl, Propionyl, Butyryl, Hexanoyl, Acryloyl, Crotonoyl, Propioloyl, und können weiter substituiert sein durch Halogen, vorzusgweise Chlor, Brom, Fluor, durch NH₂, und/oder durch -NH(C₁-C₆-Alkyl), vorzugsweise -NH(C₁-C₄-Alkyl), beispielsweise Methyl- oder Ethylamino. Aromatische Acylgruppen -NH[-C(=O)-(C₆-C₁₄-Aryl)] sind beispielsweise N-Benzoyl oder N-Naphthoyl, und können weiter substituiert sein durch Halogen, vorzugsweise Chlor, Brom, Fluor, durch C₁-C₆-Alkyl, vorzugsweise C₁-C₄-Alkyl, beispielsweise Methyl, durch Hydroxy-, durch -NH(C₁-C₆-Alkyl), vorzugsweise -NH(C₁-C₄-Alkyl), beispielsweise Methyl- oder Ethylamino, oder -O-C₁-C₆-Alkyl, vorzugsweise -O-C₁-C₄-Alkyl, beispielsweise Methoxy.

Halogen bedeutet ein Element der 7. Hauptgruppe des Periodensystems, vorzugsweise Chlor, Brom, Fluor.

R¹ ist vorzugsweise H.
R² ist vorzugsweise C₆-C₁₄-Aryl, besonders bevorzugt C₆-C₁₀-Aryl, speziell bevorzugt Phenyl, unsubstituiert oder substituiert durch (R³)ₙ. Besonders bevorzugt ist
R² Phenyl oder ein 3,4-Dihydroxyphenyl-Rest.
R³ ist vorzugsweise OH.
n ist vorzugsweise 1 oder 2.
X¹ ist vorzugsweise =CH₂.
X², X³ und X⁴ sind vorzugsweise =O.

Die allgemeinen Definitionen der Reste und die bevorzugten Definitionen der Reste R¹, R², R³, X¹, X², X³, X⁴ und n können unabhängig voneinander beliebig miteinander kombiniert werden.
Tautomere Formen der Verbindung der Verbindung (I) sind beispielweise Verbindungen der Formel (I-A) 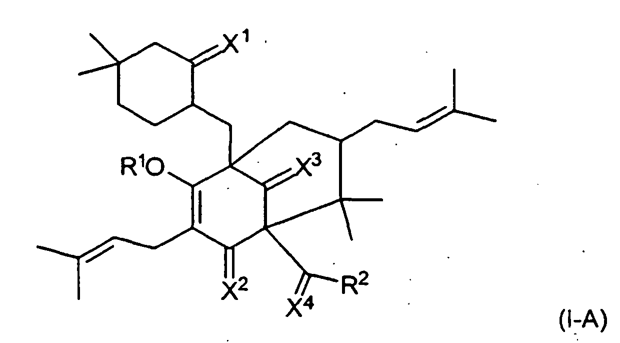 wobei die Reste R¹, R², X¹, X², X³ und X⁴ wie oben definiert sind.

Die Erfindung betrifft vorzugsweise eine Verbindung der Formel (II), 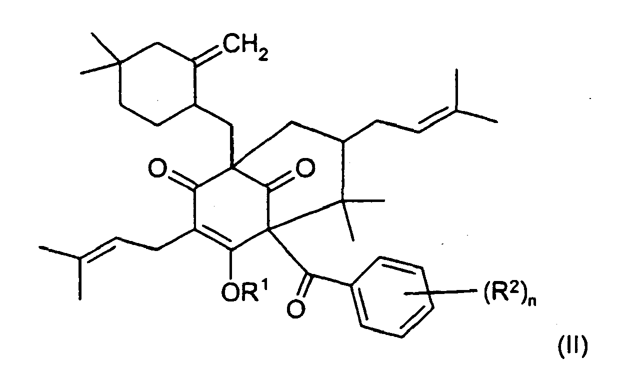 wobei R¹, R² und n die oben genannten Bedeutungen haben.

Besonders bevorzugt betrifft die Erfindung eine Verbindung der Formel (III), die im folgenden auch als Makandechamon bezeichnet wird:

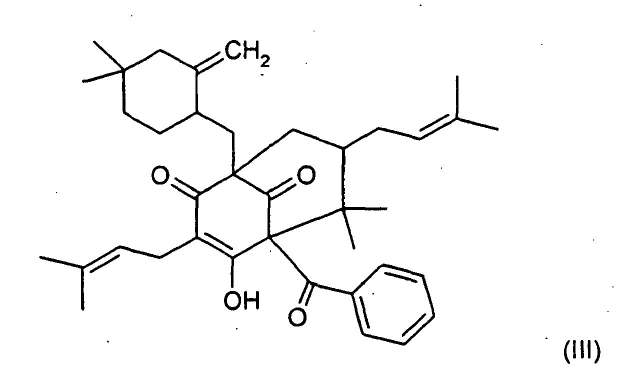

Stereoisomere Form bedeutet Enantiomer, Diastereomer und/oder Tautomer. Chiralitätszentren in den Verbindungen der Formel (I), (I-A), (II) und (III) können in der R- oder in der S-Konfiguration vorliegen. Die Erfindung betrifft sowohl die optisch reinen Verbindungen als auch Stereoisomerenjemische, wie Enantiomerengemische und Diasteromerengemische, in jedem Verhältnis.

Die erfindungsgemäßen Verbindungen unterscheidet sich von literaturbekannten Substanzen beispielsweise durch die Polarität, ihre chemische Struktur oder ihre antimikrobiellen Wirksamkeit oder weitere physikalischen Eigenschaften.

Die Erfindung betrifft desweiteren Makandechamon; eine Verbindung der Summenformel C₃₈H₅₀O₄, nachgewiesen durch ESI-Spektroskopie, und charakterisiert durch die ¹H-NMR- und ¹³C-NMR-Daten gemäß Tabelle 2 (siehe unten), oder eine stereoisomere Form der Verbindung Makandechamon oder ein Gemisch der jeweiligen vorgenannten Formen in jedem Verhältnis, oder ein physiologisch verträgliches Salz der Verbindung Makandechamon oder einer stereoisomeren Form der Verbindung Makandechamon.

Ferner betrifft die Erfindung eine Verbindung der Summenformel C₃₈H₅₀O₄ (Makandechamon) erhältlich durch Extraktion von Blättern der Pflanze Garcinia punctata oder eine Variante und/oder Mutante von Garcinia punctata und anschließender Isolierung, sowie gegebenenfalls Überführung in ein pharmakologisch verträgliches Salz.

Weiterhin betrifft die Erfindung ein Verfahren zur Herstellung der Verbindung der Formel (I) oder eines pharmakologisch verträglichen Salzes der Verbindung der Formel (I), dadurch gekennzeichnet, dass
(a) Teile der Pflanze Garcinia punctata oder eine Variante und/oder Mutante von Garcinia punctata extrahiert wird,
(b) eine Verbindung der Formel (III) isoliert und gegebenenfalls gereinigt wird,
(c) die Verbindung der Formel (III) gegebenenfalls zu einer Verbindung der Formel (I) derivatisiert wird, und
(d) die Verbindung der Formel (I) gegebenenfalls in ein pharmakologisch verträgliches Salz überführt wird.

Weiterhin betrifft die Erfindung ein Verfahren zur Herstellung der Verbindung der Formel (III) oder eines pharmakologisch verträglichen Salzes der Verbindung der Formel (III), dadurch gekennzeichnet, dass
(a) Teile der Pflanze Garcinia punctata oder eine Variante und/oder Mutante von Garcinia punctata extrahiert wird,
(b) eine Verbindung der Formel (III) isoliert und gegebenenfalls gereinigt wird,
(c) die Verbindung der Formel (III) gegebenenfalls in ein pharmakologisch verträgliches Salz überführt wird.

Vorzugsweise wird Makandechamon der Formel (III) nach ansich bekannten Methoden zu einer Verbindung der Formel (I) verestert und/oder einfach oder mehrfach hydriert. Bei Veresterungen kann die Hydroxygruppe von Makandechamon, die Teil eines vinylogen Esters ist, beispielsweise mit einem Alkylierungsmittel, wie z.B. Diazomethan oder Trimethylsilyldiazomethan verestert. Hydrierungen von werden einer oder mehreren Doppelbindungen und/oder Carbonylgruppen der Verbindung der Formel (I) können mit einem Reduktionsmittel reduziert werden, wobei Doppelbindungen zum Beispiel mit H₂/Pd und Carbonylgruppen zum Beispiel mit NaBH₄ reduziert werden. Die oben genannten Methoden zur Derivatisierung sind in Lehrbüchern wie Jerry March, Advanced Organic Chemistry, John Wiley & Sons, 4th Edition, 1992, beschrieben. Um Umsetzungen selektiv durchzuführen, kann es vorteilhaft sein, in ansich bekannter Weise vor der Reaktion geeignete Schutzgruppen einzuführen. Die Schutzgruppen können nach der Reaktion abgespalten werden.

Die Extraktion der erfindungsgemäßen Verbindungen kann entsprechend dem Fachmann bekannten Methoden, wie z.B. durch Austestung der biologischen Aktivität in Bioassays oder durch chromatographische Methoden wie Dünnschichtchromatographie (DC) oder Hochleistungsflüssigkeits-Chromatographie (HPLC) verfolgt werden.

Garcinia punctata ist ein immergrüner Baum aus der Familie der Clusiaceae. Verbreitungsgebiet der Clusiaceae sind die gemäßigten und tropischen Regionen, es finden sich zahlreiche tropische Heil- und Nutzpflanzen in dieser Familie. Die Verbreitung von Garcinia punctata beschränkt sich im wesentlichen auf die westafrikanische Region, die mit der Nummer PLA 100848 versehene Pflanze von Garcinia punctata wurde in Gabun in unmittelbarer Nähe der Station La Makande Research Field Station (Koordinaten 0° 40' 860" S - 11 ° 54' 750" E) gesammelt. Garcinia punctata besitzt einfache gegenständige Blätter und enthält orange bis rot gefärbten Latex. Die Blüten sind gelb oder weiß. Die Probe PLA 100848, aus der die Verbindung Makandechamon isoliert wurde, stammt aus dem Bereich der Baumkrone.

Das Screening nach Mutanten und Varianten, die das erfindungsgemäße Antibiotikum produzieren, kann durch Bestimmung der biologischen Aktivität in den Extrakten angehäuften Wirkstoffes, beispielsweise durch Bestimmung der antibakteriellen Wirkung erfolgen, oder durch Detektion von Verbindungen, die als antibakteriell aktiv bekannt sind, in den Extrakten durch beispielsweise HPLC -oder LC-MS-Methoden.

Das besagte Verfahren umfasst die Extraktion von allen Teilen der Pflanze Garcina punctata, vorzugsweise den Blättern. Die Verbindung Makandechamon kommt insbesondere in den Blättern der Pflanze Garcinia punctata vor, und wird vorzugsweise aus getrockneten Blättern gewonnen. Zur Familie der Clusiaceae gehören zahlreiche Heil- und Nutzpflanzen. Die Pflanzen sind in den gemäßigten und tropischen Klimaten weit verbreitet.

Die getrockneten und fein gemahlenen Blätter werden mit einem organischen Lösemittel, zum Beispiel Methanol oder Propanol-2 extrahiert, optional im Gemisch mit Wasser oder einem wässrigen Puffer.

Die Extraktion kann in einem weiten pH -Bereich durchgeführt werden, es ist jedoch zweckmäßig, im neutralen oder schwach sauren Milieu, vorzugsweise zwischen pH 3 und pH 7 zu arbeiten. Der Extrakt kann z.B. im Vakuum konzentriert und getrocknet werden.

Zur Isolierung von Makandechamon können auch Pflanzen derselben Gattung verwendet werden, die an einem anderen Standort gesammelt wurden. Der Gehalt an Makandechamon kann ja nach Standortverhältnissen, wie z.B. Bodenbeschaffenheit, Lichteinfall, Temperatur, Feuchtigkeit oder Lichteinfall variieren.

Das erfindungsgemäße Verfahren kann für die Extraktion und Isolierung im Labormaßstab (100 g bis 1 kg trockene Pflanzenmasse) und im industriellen Maßstab (100 bis > 1000 kg) eingesetzt werden.

Die Kultivierung der Pflanzen kann im Freiland oder vorzugsweise im Gewächshaus erfolgen, alternativ können pflanzliche Zellkulturen zur Produktion der Metabolite eingesetzt werden. In der Regel kultiviert man dazu in mehreren Stufen, d.h. man stellt zunächst eine oder mehrere Vorkulturen in einem geeigneten Flüssigmedium her, mit denen dann die Hauptkultur, beimpft werden kann. Das Ausgangsmaterial besteht in der Regel aus Kalluskulturen. Durch die Auswahl geeigneter Bioreaktoren zur Züchtung der pflanzlichen Zellkultur kann eine optimale Durchmischung und Belüftung der Kultur ohne die Einwirkung von zu starken Scherkräften auf die Pflanzenzellen und damit optimales Zellwachstum und Metabolit-Produktion erzielt werden. Beispielsweise können Airlift- oder Blasensäulenreaktoren, sowie Blatt- oder Propellerrührwerke zur Durchmischung der Kulturen eingesetzt werden. Die Zellen können als Einzelzellen bzw. verzweigte oder unverzweigte Zell-Aggregate oder -ketten wachsen. Die Metabolitproduktion kann durch Stimulation mit exogenen Faktoren, z.B. Schwermetallsalzen oder pflanzlichen Elicitoren induziert werden.

Die Produktbildung in der Pflanzenzellkultur kann anhand des pH-Wertes der Kulturen sowie durch chromatographische Methoden, wie z.B. Dünnschichtchromatographie, HPLC oder Ausprüfen der biologischen Aktivität überwacht werden. Die erfindungsgemäße Verbindung Makandechamon kann neben den Blättern auch in anderen Pflanzenteilen enthalten sein. Das im folgenden beschriebene Isolierungsverfahren dient zur Aufreinigung der erfindungsgemäßen Verbindung Makandechamon.

Die Isolierung bzw. Aufreinigung der erfindungsgemäßen Verbindungen,aus der Pflanze oder dem Kulturmedium erfolgt nach bekannten Methoden unter Berücksichtigung der chemischen, physikalischen und biologischen Eigenschaften der Naturstoffe. Zum Testen der Konzentration der gewünschten Verbindung im Ausgangsmaterial oder in den einzelnen Isolierungsstufen kann die HPLC verwendet werden, wobei die Menge der gebildeten Substanz zweckmäßig mit einer Eichlösung verglichen wird.

Zur Isolierung von Makandechamon werden die Blätter der Pflanze geerntet und noch in frischem Zustand oder getrocknet nach den üblichen Verfahren extrahiert und anschließend wird Makandechamon aus dem Pflanzenmaterial mit einem gegebenenfalls wasserhaltigem organischen Lösungsmittel extrahiert. Die organische Lösungsmittelphase enthält das erfindungsgemäße Makandechamon, es wird gegebenenfalls im Vakuum konzentriert und weiter aufgereinigt.

Eine weitere Methode der Reinigung ist die Chromatographie an Adsorptionsharzen wie z.B. an Diaion® HP-20 (Mitsubishi Casei Corp., Tokyo), an Ambertite® XAD 7. (Rohm and Haas, USA), an Amberchrom® CG, (Toso Haas, Philadelphia, USA) oder an ähnlichen. Geeignet sind darüber hinaus zahlreiche Reversed- Phase Träger, z.B. RP₈ und RP₁₈, wie sie z.B. im Rahmen der Hochdruckflüssigkeits-Chromatographie (HPLC) allgemein bekannt geworden sind.

Eine weitere Reinigungsmöglichkeit für die erfindungsgemäße Verbindung besteht in der Verwendung von sogenannten Normal-Phasen-Chromatographie-Trägem, wie z.B. Kieselgel oder Al₂O₃ oder anderen in an sich bekannter Weise.
Ein alternatives Isolierungsverfahren ist die Verwendung von Molekularsieben, wie z.B. Fractogel® TSK HW-40 (Merck, Deutschland) und andere, in an sich bekannter Weise. Es ist darüber hinaus, auch möglich, aus angereichertem Material das Makandechamon durch Kristallisation zu gewinnen. Geeignet hierzu sind z.B. organische Lösemittel und ihre Gemische, wasserfrei oder mit Wasserzusatz. Ein zusätzliches Verfahren zur Isolierung und Reinigung der erfindungsgemäßen Antibiotika besteht in der Verwendung von Anionenaustauschern, vorzugsweise im pH-Bereich von 4 bis 10 und Kationenaustauschern vorzugsweise im pH-Bereich von 2 bis 5. Besonders geeignet ist hierfür die Verwendung von Pufferlösungen, denen man Anteile von organischen Lösungsmitteln hinzugefügt hat.

Die Verbindungen der Formel (I), (II) und (III) können nach dem Fachmann bekannten Methoden in pharmakologisch verträgliche Salze überführt werden. Unter pharmakologisch verträglichen Salzen der erfindungsgemäßen Verbindungen versteht man sowohl anorganische als auch organische Salze, wie sie in Remingtons Pharmaceutical Sciences (17. Auflage, Seite 1418 [1985]) beschrieben sind. Als Salze kommen insbesondere Alkali-, Ammonium-, Erdalkalisalze, Salze mit physiologisch verträglichen Aminen und Salze mit anorganischen oder organischen Säuren wie z.B. HCl, HBr, H₂SO₄, Maleinsäure, Fumarsäure in Frage.

Es wurde überraschend gefunden, dass die erfindungsgemäßen Verbindungen der Formel (I) antibakterielle Wirkungen aufweisen, insbesondere gegen humanpathogene Keime, und sich daher zur Behandlung und/oder Prophylaxe von Erkrankungen eignen, die durch bakterielle Infektionen verursacht werden.

Die vorliegende Erfindung betrifft daher auch die Verwendung einer oder mehrerer der erfindungsgemäßen Verbindungen der Formel (I), (II) und/oder (III) als Arzneimittel, insbesondere zur Behandlung und/oder Prophylaxe von bakteriellen Infektionen.

Ferner betrifft die Erfindung die Verwendung einer oder mehrerer der erfindungsgemäßen Verbindungen der Formel (I), (II) und/oder (III) zur Herstellung von Arzneimitteln, insbesondere zur Behandlung und/oder zur Prophylaxe von bakteriellen Infektionen.
Des weiteren betrifft die vorliegende Erfindung ein Arzneimittel mit einem Gehalt an mindestens einer Verbindung der Formel (I), (II) und/oder (III).

Das besagte Arzneimittel enthaltend eine Verbindung der Formel (I), (II) und/oder (III) wird mit einem oder mehreren physiologisch geeigneten Hilfsstoffen hergestellt und in eine geeignete Darreichungsform gebracht.

Die erfindungsgemäßen Arzneimittel können enteral (oral), parenteral (intramuskulär oder intravenös), rektal oder lokal (topisch) angewendet werden. Sie können in Form von Lösungen, Pulvern (Tabletten, Kapseln einschließlich Mikrokapseln), Salben (Cremes oder Gel), oder Suppositorien verabreicht werden. Als physiologisch geeignete Hilfsstoffe für derartige Formulierungen kommen die pharmazeutisch üblichen flüssigen oder festen Füllstoffe und Streckmittel, Lösemittel, Emulgatoren, Gleitstoffe, Geschmackskorrigentien, Farbstoffe und/oder Puffersubstanzen in Frage. Als zweckmäßige Dosierung werden 0.1 - 1000, vorzugsweise 0.2 - 100 mg/kg Körpergewicht verabreicht. Sie werden zweckmäßig in Dosierungseinheiten verabreicht, die mindestens die wirksame Tagesmenge der erfindungsgemäßen Verbindungen, z.B. 30 - 3000, vorzugsweise 50 - 1000 mg enthalten.

Die folgenden Beispiele sollen der näheren Erläuterung der Erfindung :dienen, ohne die Breite der Erfindung in irgendeiner Weise zu begrenzen.

### Beispiel 1 Herstellung des Rohextraktes der Pflanze Garcinia punctata, PLA-100848

Blätter von Garcinia punctata, PLA-100848, wurden im frischen Zustand gesammelt und bei maximal 40°C luftgetrocknet. 100 g Trockenmaterial wurden fein gemahlen und mit 1 L Methanol für mindestens 8h unter Rühren extrahiert. Der Extrakt wurde abfiltriert und anschließend unter Vakuum bis nahezu zur Trockene aufkonzentriert. Der Rückstand wurde mit Wasser resusupendiert und gefriergetrocknet. Der so hergestellte Primärextrakt kann bei 4°C oder-20°C aufbewahrt werden oder zur weiteren Isolierung wie in Beispiel 2 beschrieben verwendet werden. Zum Testen der biologischen Aktivität wurden zunächst aus dem Primärextrakt mittels Chromatographie an Polyamid und Polystyrol-Adsorberharz Tannine sowie andere stark hydrophile bzw. lipophile Störsubstanzen entfernt.

### Beispiel 2: Isolierung und Reinigung der Verbindung Makandechamon.

1 g des angereicherter Rohextrakt von Makandechamon, gewonnen nach Beispiel 1, wurden in 5 mL Methanol gelöst und zentrifugiert und der Überstand auf einer LUNA® 10 µm C 18(2)-HPLC-Säule (Phenomenex, USA) (Breite x Höhe = 2.1 cm x 25 cm) im Gradientenverfahren mit 5 % bis 95 % Acetonitril in 0.1 % Ammoniumacetat pH 4,5 aufgetrennt. Fluß: 33 mL/Min. Fraktionsgröße: 33 mL. Die durch analytische HPLC (siehe Beispiel 3) untersuchten Fraktionen 44 und 45 wurden gesammelt und gefriergetrocknet. Sie ergaben 15 mg Makandechamon in 95%iger Reinheit.

### Beispiel 3: Pflanzenproduktion, Sammeln der Samen, Aussaat und Wachstumssowie Produktionsbedingungen

Die Samen von Garcinia punctata, PLA 100848, wurden nach der Reifung gesammelt und zur weiteren Anzucht der Pflanzen im Gewächshaus ausgesäat. Die optimale Temperatur betrug ca. 28° C bei einer Luftfeuchtigkeit von 70-90%. Die Pflanzen wurden mehrere Monate bis Jahre kultiviert, bis zur Ernte der Blätter oder anderer geeigneter Pflanzenteile.

### Beispiel 4: Analytische HPLC

- Säule:: Purospher ® STAR RP-18 e 3 µm, 30-2, (Merck, Deutschland)
- Mobile Phase Puffer A:: 5 % Acetonitril + 0.1% Ammoniumacetat,
- Mobile Phase Puffer B:: 95 % Acetonitril + 0.1% Ammoniumacetat,
- Gradient:: 15 min
- Flußgeschwindigkeit:: 0.25 mL pro Minute
Detektion durch UV-Absorption bei 210 nm.

Für Makandechamon wurde eine Retentionszeit von 8.9 Min. gefunden.

### Beispiel 5: Charakterisierung von Makandechamon.

Die physikalisch-chemischen sowie spektroskopischen Eigenschaften des erfindungsgemäßen Antibiotikums lassen sich wie folgt zusammenfassen:

### Aussehen:

Farblose bis hellgelbe, in mittelpolaren und polaren organischen Lösungsmitteln lösliche, in Wasser wenig lösliche Substanz. Stabil in neutralem und saurem Milieu.
- Summenformel:: C₃₈H₅₀O₄
- Molekulargewicht:: 570,82
- ¹H- und ¹³C-NMR:: siehe Tabelle 1
- UV-Maxima:: 240 nm, 290 nm

### Bestimmung des Molpeaks:

Dem gesuchten Molekül wird die Masse 570 zugeordnet aufgrund folgender Befunde: ESI⁺-Spektrum zeigt einen Peak bei 571 amu (M+H)⁺,
MS/MS ESI^{+:} 447 amu (M+H)⁺, 435 amu, 379 amu, 311 amu, 255 amu, 177 amu, 105 amu

Hochauflösung des Quasi-Molekülions: ESI⁺ 571,3783 (M+H)⁺. Für die Summenformel C₃₈H₅₀O₄ wurde 5713782, berrechnet.

**Tabelle 1: ¹H- und ¹³C-NMR-chemische Verschiebungen von Makandechamon in MeOD bei 303 K.**

| 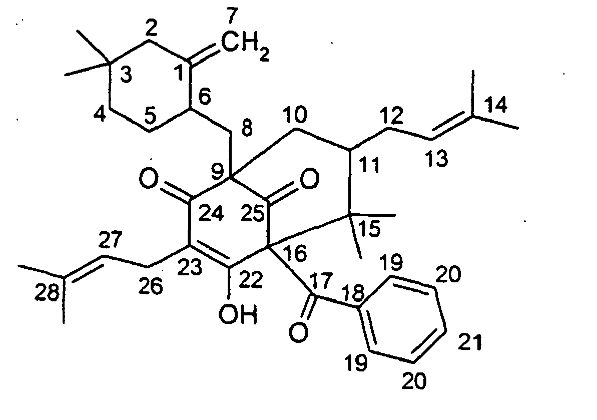 | | |
|---|---|---|
| | ¹H | ¹³C |
| 1 | - | 151.79 |
| 2 | 2.04/1:66 | 49.0 |
| 3 | - | 33.52 |
| 3-Me | 0.84 | 29.20 |
| 3-Me' | 0.83 | 28.31 |
| 4 | 1.49/1.17 | 38.59 |
| 5 | 1.55/1.31 | 31.18 |
| 6 | 2.37 | 40.04 |
| 7 | 4.62/4.53 | 108.31 |
| 8 | 2.10/1.98 | 34.59 |
| 9 | - | ~61.5 a) |
| 10 | 2.00/1.42 | 45.06 |
| 11 | 1.70 | 44.11 |
| 12 | 2.11/1.71 | 28.31 |
| 13 | 4.99 | 124.00 |
| 14 | - | 134.17 |
| 14-Me | 1.68 | 25.98 |
| 14-Me' | 1.58 | 17.97 |
| 15 | - | - 49.0 |
| 15-Me | 1.34 | 24.56 |
| 15-Me' | 1.10 | 16.23 |
| 16 | - | ~77.6 a) |
| 17 | - | 195.20 |
| 18 | - | 138.70 |
| 19 | 7.64 | 129.63 |
| 20 | 7.28 | 128.83 |
| 21 | 7.46 | 133.05 |
| 22 | - | b) |
| 23 | - | 120.30 |
| 24 | - | b) |
| 25 | - | 209.88 |
| 26 | 3.20/3.07 | 22.42 |
| 27 | 5.09 | 121.95 |
| 28 | - | 133.46 |
| 28-Me | 1.68 | 18.15 |
| 28-Me' | 1.66 | 26.09 |

| | | |
|---|---|---|
| a) Für dieses C-Atom wird im ¹³C-Spektrum kein Signal beobachtet. Die Zuordnung erfolgte anhand von Korrelationen im HMBC-Spektrum. b) Für dieses C-Atom wird im ¹³C-Spektrum kein Signal beobachtet. | | |

### Beispiel 6: Test der antibakteriellen Aktivität

Für einen Test der antibakteriellen Wirkung wurden zunächst Agarplatten mit 2 mL *Staphylococcus aureus* -Einsaat in 200 mL Agarlösung vorbereitet. Makandechamon wurde in einer 1 mg/ml Lösung auf Antibiotika-Testblättchen (Schleicher und Schüll) mit einem Durchmesser von 6 mm aufgetragen und auf die Agarplatte gelegt. Die beimpften Staphylococcus-Platten wurden 16 Stunden bei 37°C inkubiert. Dann wurden Hemmhöfe mit folgenden Durchmessern (mm) beobachtet:

| | |
|---|---|
| Menge | Hemmhofgröße (mm) |
| 10 µL | 7 |
| 20 µL | 12 |
| 40 µL | 14 |

## Patentansprüche

1. . Verbindung der Formel (I) 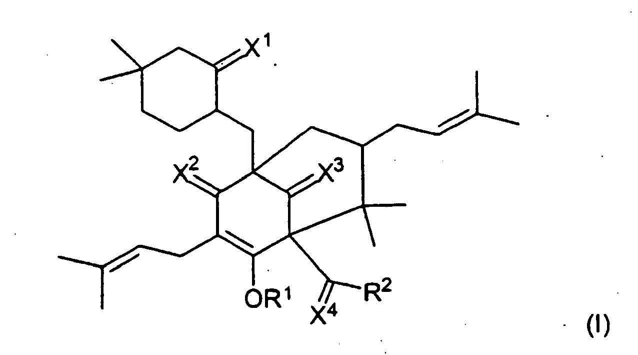 wobei
R¹
H, C₁-C₆-Alkyl, C₂-C₆-Alkenyl, C₂-C₆-Alkinyl, oder C₆-C₁₄-Aryl bedeutet,
worin Alkyl, Alkenyl, Alkinyl und Aryl unsubstituiert oder ein- bis dreifach durch einen Rest R³ substituiert sind,
R²
C₁-C₆-Alkyl, C₂-C₆-Alkenyl, C₂-C₆-Alkinyl, oder C₆-C₁₄-Aryl bedeutet,
worin Alkyl, Alkenyl, Alkinyl und Aryl unsubstituiert oder n-fach durch einen Rest R³ substituiert sind, wobei n eine ganze Zahl von 1 bis 3 ist, und
R³
-OH, =O, -O-C₁-C₆-Alkyl, -O-C₂-C₆-Alkenyl, -O-C₆-C₁₄-Aryl, -NH-C₁-C₆-Alkyl. -NH-C₂-C₆-Alkenyl, -NH[-C(=O)-(C₁-C₆-Alkyl)], -NH[-C(=O)-(C₆-C₁₄-Aryl)], -NH₂
oder Halogen bedeutet, wenn R¹ und R² unabhängig voneinander Alkyl, Alkenyl oder Alkinyl sind, worin Alkyl, Alkenyl und Alkinyl weiter substituiert sein können durch -CN, -Amid oder -Oxim-Funktionen, oder
-OH, -O-C₁-C₆-Alkyl, -O-C₂-C₆-Alkenyl, -O-C₆-C₁₄-Aryl, -NH-C₁-C₆-Alkyl, -NH-C₂-C₆-Alkenyl, -NH[-C(=O)-(C₁-C₆-Alkyl)], -NH[-C(=O)-(C₆-C₁₄-Aryl)], -NH₂ oder Halogen bedeutet, wenn R¹ oder R² Aryl sind, und worin Aryl weiter substituiert sein kann durch -CN oder -Amid,
X¹
=CH₂ oder =O bedeutet,
X², X³ und X⁴ unabhängig voneinander
=O, =NR¹ oder =S bedeutet,
oder eine stereoisomere Form der Verbindung der Formel (I) oder ein Gemisch von Stereoisomeren einer Verbindnung der Formel (I) in jedem Verhältnis, oder ein physiologisch verträgliches Salz einer Verbindung der Formel (I) oder ein physiologisch verträgliches Salz einer stereoisomeren Form einer Verbindung der Formel (I).

2. . Verbindung der Formel (I) gemäß Anspruch 1, **gekennzeichnet durch** eine Verbindung der Formel (II)

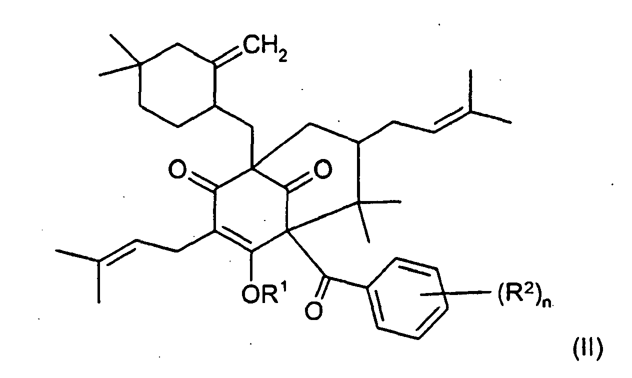

3. . Verbindung der Formel (I) gemäß einem der Ansprüche 1 bis 2, **gekennzeichnet durch** eine Verbindung der Formel (III)

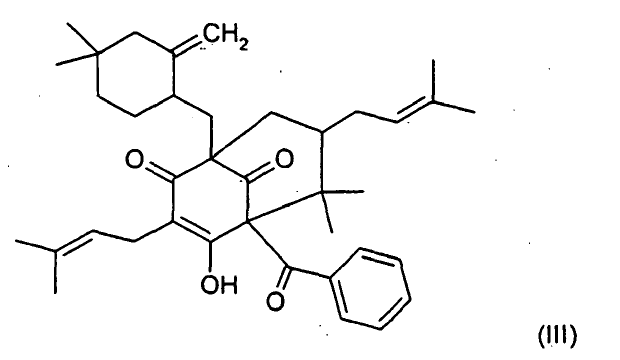

4. . Verfahren zur Herstellung einer Verbindung der Formel (I) gemäß einem oder mehreren der Ansprüche 1 bis 3, **dadurch gekennzeichnet, daß**
(a) Teile der Pflanze Garcinia punctata oder eine ihrer Varianten und/oder Mutanten extrahiert wird,
(b) eine Verbindung der Formel (III) isoliert und gegebenfalls gereinigt wird,
(c) die Verbindung der Formel (III) gegebenenfalls mit einem geeigneten Reagenz in eine Verbindung der Formel (I) derivatisiert wird,
(d) und die Verbindung der Formel (1) gegebenenfalls in ein pharmakologisch verträgliches Salz überführt wird.

5. . Verfahren zur Herstellung einer Verbindung der Formel (III) gemäß Anspruch 3,
**dadurch gekennzeichnet, daß**
(a) Teile der Pflanze Garcinia punctata oder eine ihrer Varianten und/oder Mutanten extrahiert wird,
(b) eine Verbindung der Formel (III) isoliert und gegebenfalls gereinigt wird, und
(c) die Verbindung der Formel (III) gegebenenfalls in ein pharmakologisch verträgliches Salz überführt wird.

6. . Verwendung einer Verbindung gemäß einem oder mehreren der Ansprüche 1 bis 3 zur Herstellung eines Arzneimittels.

7. . Verwendung einer Verbindung gemäß einem der Ansprüche 1 bis 3 zur Herstellung eines Arzneimittels zur Behandlung und/oder Prophylaxe von bakteriellen Infektionen.

8. . Arzneimittel mit einem Gehalt an mindestens einer Verbindung gemäß einem oder mehreren der Ansprüche 1 bis 3 und einem oder mehreren physiologisch geeigneten Hilfsstoffen.

9. . Verfahren zur Herstellung eines Arzneimittels gemäß Anspruch 8, **dadurch gekennzeichnet, daß** mindestens eine Verbindung gemäß einem oder mehreren der Ansprüche 1 bis 3 mit einem oder mehreren physiologisch geeigneten Hilfsstoffen in eine geeignete Darreichungsform gebracht wird.

## Claims

1. A compound of the formula (I) 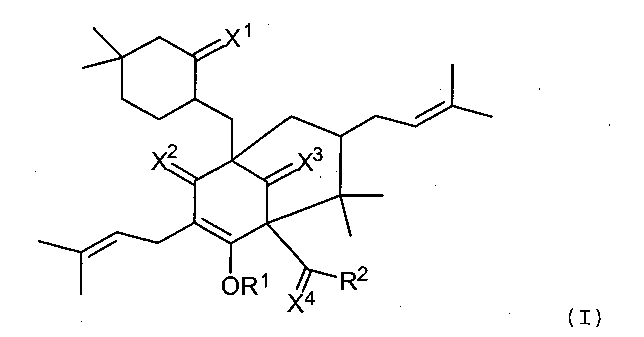 where
R¹
is H, C₁-C₆-alkyl, C₂-C₆-alkenyl, C₂-C₆-alkynyl, or C₆-C₁₄-aryl,
in which alkyl, alkenyl, alkynyl and aryl are unsubstituted or mono- to tri-substituted by a radical R³,
R²
is C₁-C₆-alkyl, C₂-C₆-alkenyl, C₂-C₆-alkynyl, or C₆-C₁₄-aryl,
in which alkyl, alkenyl, alkynyl and aryl are unsubstituted or substituted n times by a radical R³, where n is an integer from 1 to 3, and
R³
is -OH, =0, -0-C₁-C₆-alkyl, -O-C₂-C₆-alkenyl, -O-C₆-C₁₄-aryl, -NH-C₁-C₆-alkyl, -NH-C₂-C₆-alkenyl, -NH[-C(=O)-(C₁-C₆-alkyl)], -NH[-C(-O)-(C₆-C₁₄-aryl)], -NH₂ or halogen, when R¹ and R², independently of one another, are alkyl, alkenyl or alkynyl, in which alkyl, alkenyl and alkynyl can be further substituted by -CN, -amide or -oxime functions, or
is -OH, -O-C₁-C₆-alkyl, -O-C₂-C₆-alkenyl, -O-C₆-C₁₄-aryl, -NH-C₁-C₆-alkyl, -NH-C₂-C₆-alkenyl, -NH[-C(=O)-(C₁-C₆-alkyl)], -NH[-C(=O)-(C₆-C₁₄-aryl)], -NH₂ or halogen, when R¹ or R² are aryl, and in which aryl can be further substituted by -CN or -amide,
X¹
are =CH₂ or =O,
X², X³ and X⁴ independently of one another
is =O, =NR¹ or =S,
or a stereoisomeric form of the compound of the formula (I) or a mixture of stereoisomers of a compound of the formula (I) in any ratio, or a physiologically tolerable salt of a compound of the formula (I) or a physiologically tolerable salt of a stereoisomeric form of a compound of the formula (I).

2. A compound of the formula (I) as claimed in claim 1, comprising a compound of the formula (II)

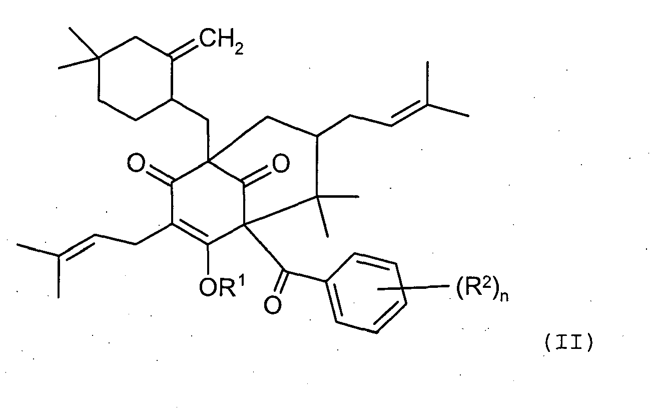

3. A compound of the formula (I) as claimed in either of claims 1 and 2, comprising a compound of the formula (III)

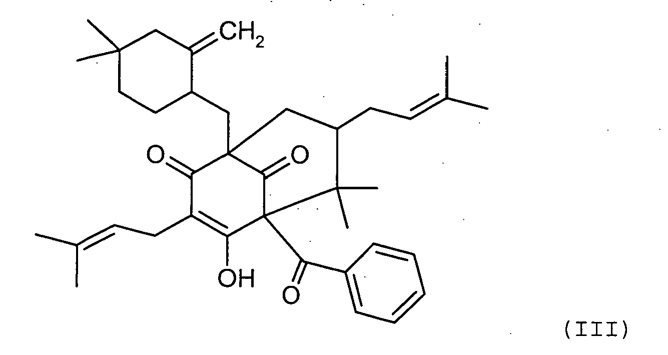

4. A process for the preparation of a compound of the formula (I) as claimed in one or more of claims 1 to 3, which comprises
(a) extracting parts of the plant Garcinia punctata or one of its variants and/or mutants,
(b) isolating and optionally purifying a compound of the formula (III),
(c) derivatizing the compound of the formula (III), if appropriate using a suitable reagent, to give a compound of the formula (I),
(d) and converting the compound of the formula (I), if appropriate, into a pharmacologically tolerable salt.

5. A process for the preparation of a compound of the formula (III) as claimed in claim 3, which comprises
(a) extracting parts of the plant Garcinia punctata or one of its variants and/or mutants,
(b) isolating and optionally purifying a compound of the formula (III), and
(c) converting the compound of the formula (III), if appropriate, into a pharmacologically tolerable salt.

6. The use of a compound as claimed in one or more of claims 1 to 3 for the production of a pharmaceutical.

7. The use of a compound as claimed in any of claims 1 to 3 for the production of a pharmaceutical for the treatment and/or prophylaxis of bacterial infections.

8. A pharmaceutical containing at least one compound as claimed in one or more of claims 1 to 3 and one or more physiologically suitable excipients.

9. A process for the production of a pharmaceutical as claimed in claim 8, which comprises bringing at least one compound as claimed in one or more of claims 1 to 3 into a suitable administration form using one or more physiologically suitable excipients.

## Revendications

1. Composé de formule (I) 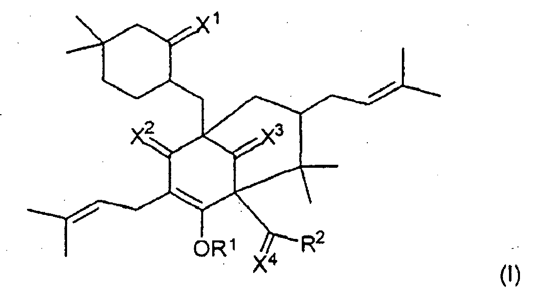 où
R¹ signifie H, C₁-C₆-alkyle, C₂-C₆-alcényle, C₂-C₆-alcynyle, - ou C₆-C₁₄-aryle,
où alkyle, alcényle, alcynyle et aryle sont non substitués ou monosubstitués à trisubstitués par un radical R³,
R² signifie C₁-C₆-alkyle, C₂-C₆-alcényle, C₂-C₆-alcynyle, ou C₆-C₁₄-aryle,
où alkyle, alcényle, alcynyle et aryle sont non substitués ou n-substitués par un radical R³, où n est un nombre entier de 1 à 3 et
R³ signifie -OH, =O, -O-C₁-C₆-alkyle, -O-C₂-C₆-alcényle, -O-C₆-C₁₄-aryle, -NH-C₁-C₆-alkyle, -NH-C₂-C₆-alcényle, -NH[-C(=O)-(C₁-C₆-alkyle)],
-NH[-C(=O)-(C₆-C₁₄-aryle)], -NH₂ ou halogène, lorsque R¹ et R² représentent, indépendamment l'un de l'autre, alkyle, alcényle ou alcynyle, où alkyle, alcényle et alcynyle peuvent en outre être
substitués par des fonctions -CN, -amide ou -oxime, ou
signifie -OH, -O-C₁-C₆-alkyle, -O-C₂-C₆-alcényle, -O-C₆-C₁₄-aryle, -NH-C₁-C₆-alkyle, -NH-C₂-C₆-alcényle, -NH[-C(=O)-(C₁-C₆-alkyle)], -NH [-C(=O)-(C₆-C₁₄-aryle)], -NH₂ ou halogène, lorsque R¹ et R² représentent aryle et où aryle peut en outre être substitué par des fonctions -CN ou -amide,
X¹ signifie =CH₂ ou =O,
X², X³ et X⁴ signifient indépendamment l'un de l'autre, =O, =NR¹ ou =S,
ou une forme stéréo-isomère du composé de formule (I)
ou un mélange de stéréo-isomères d'un composé de formule (I) dans n'importe quel rapport, ou un sel physiologiquement acceptable d'un composé de formule (I) ou un sel physiologiquement acceptable d'une forme stéréo-isomère d'un composé de formule (I).

2. Composé de formule (I), selon la revendication 1, **caractérisé par** un composé de formule (II)

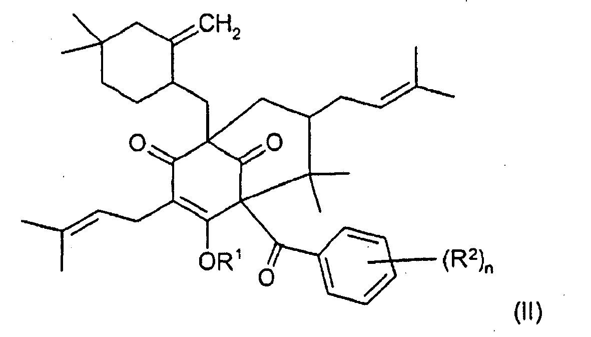

3. Composé de formule (I) selon l'une quelconque des revendications 1 à 2, **caractérisé par** un composé de formule (III)

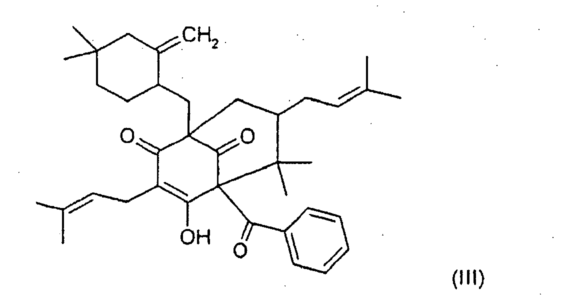

4. Procédé pour la préparation d'un composé de formule (I) selon l'une ou plusieurs des revendications 1 à 3, **caractérisé en ce qu'**on
(a) extrait des parties de la plante Garcinia punctata ou d'une de ses variantes et/ou d'un de ses mutants,
(b) isole et purifie le cas échéant un composé de formule (III),
(c) dérive le composé de formule (III) le cas échéant avec un réactif approprié en un composé de formule (I),
(d) et transforme le composé de formule (I) le cas échéant en un sel pharmacologiquement acceptable.

5. Procédé pour la préparation d'un composé de formule (III) selon la revendication 3, **caractérisé en ce qu'**on
(a) extrait des parties de la plante Garcinia punctata ou d'une de ses variantes et/ou d'un de ses mutants,
(b) isole et purifie le cas échéant un composé de formule (III), et
(c) transforme le composé de formule (III) le cas échéant en un sel pharmacologiquement acceptable.

6. Utilisation d'un composé selon l'une ou plusieurs des revendications 1 à 3 pour la préparation d'un médicament.

7. Utilisation d'un composé selon l'une quelconque des revendications 1 à 3 pour la préparation d'un médicament destiné au traitement et/ou à la prophylaxie d'infections bactériennes.

8. Médicament présentant une teneur en au moins un composé selon l'une ou plusieurs des revendications 1 à 3 et un ou plusieurs adjuvants physiologiquement appropriés.

9. Procédé pour la préparation d'un médicament selon la revendication 8, **caractérisé en ce qu'**on amène au moins un composé selon l'une ou plusieurs des revendications 1 à 3 avec un ou plusieurs adjuvants physiologiquement appropriés dans une forme d'administration appropriée.
